(19) Europäisches Patentamt
European Patent Office
Office européen des brevets

(11) Publication number: 0 009 670
A1

## (12) EUROPEAN PATENT APPLICATION

(21) Application number: 79103380.6

(22) Date of filing: 10.09.79

(51) Int. Cl.[3]: C 07 H 15/22
A 61 K 31/70
//C07D207/26, C07D207/24
C07D211/76

(30) Priority: 11.09.78 JP 112119/78

(43) Date of publication of application:
16.04.80 Bulletin 80/8

(84) Designated Contracting States:
CH DE FR GB IT NL SE

(71) Applicant: Shionogi & Co., Ltd.
12, 3-chome Dosho-machi Higashi-ku
Osaka(JP)

(72) Inventor: Igarashi, Kikuo
11-5, Nishinosotogawara
Itami-shi Hyogo Pref.(JP)

(72) Inventor: Honma, Tsunetoshi
117-42, Aoyamadai
Ikoma-shi Nara Pref.(JP)

(74) Representative: Vossius. Vossius. Hiltl. Tauchner. Heunemann et al,
Siebertstrasse 4 P.O. Box 86 07 67
D-8000 München 86(DE)

## (54) Aminoglycoside derivatives, process for their preparation and pharmaceutical compositions.

(57) Aminoglycoside derivatives of the general formula (I)

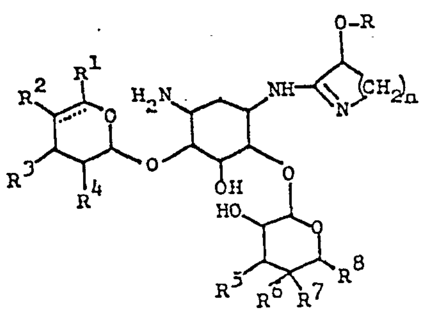

(I)

are disclosed,
wherein R is hydrogen or acyl:
n is an integer of 1 to 3;
$R^1$ is aminomethyl, hydroxymethyl, 1-aminoethyl, or 1-methylaminoethyl;
$R^2$, $R^3$ and $R^6$ are each hydrogen or hydroxy;
$R^4$ is hydroxy or amino;
$R^5$ is amino or methylamino;
$R^7$ is hydroxy or methyl;
$R^8$ is hydrogen, hydroxymethyl or carbamoyloxymethyl;
and the dotted line represents the presence or absence of a double bond, with the proviso that if the dotted line represents a double bond, $R^5$ is methylamino, and their salts with acids together with a process for their preparation and pharmaceutical composition having antibiotic activity and containing compounds of the general formula (I)

BAD ORIGINAL

EP 0 009 670 A1

Aminoglycoside antibiotics, for example, streptomycin, kanamycins, gentamicins, tobtamycin, etc. have been used in practice as broad spectrum antimicrobials effective against gram-positive, gram-negative and acid-fast bacteria. The aminoglycoside antibiotics, however, are sometimes accompanied by undesired side effects such as nephropathy and deafness. The occurrence of strains resistant to the aminoglycosides is another problem to be solved. It has been attempted to modify such aminoglycoside by acylating the 1-amino group with specific acyl groups in order to improve the antimicrobial activity and relatively decrease the side effects. For instance, amikacin which is prepared by acylation of the 1-amino group of kanamycin A with (S)-4-amino-2-hydroxybutyric acid [Kawaguchi et al, J. Antibiotic, 25, 695 (1972) ; U.S.Pat. 3,781,268 (1973) ; J, Antibiotic 27, 677 (1974)] and butyrosin which is prepared by acylating the 1-amino group of ribostamycin with (S)-4-amino-2-hydroxybutyric acid [U.S.Pat. 3,541,078], are well known. Further, an aminoglycoside derivative which is prepared by introduction of (SR)-3-hydroxy-1-pyrrolin-2-yl into the 1-amino group of ribostamycin is published in Carbohydrate Research, 28 (1973), 263 to 280.

It is an object of the invention to provide novel aminoglycoside derivatives having an excellent antimicrobial action.

This invention relates to novel aminoglycoside antibiotic derivatives represented by the general formula (I):

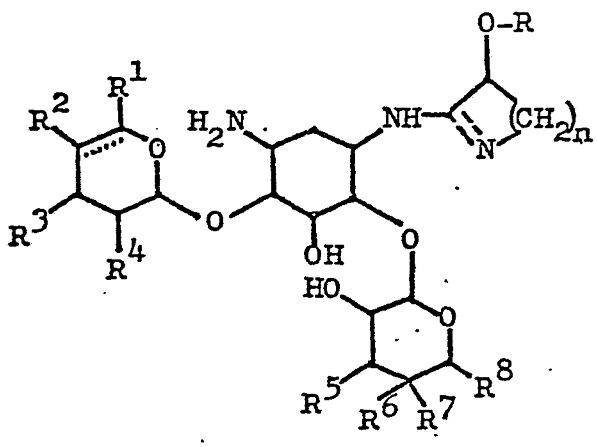

(I)

wherein R is hydrogen or acyl ;

n is an integer of 1 to 3 ;

$R^1$ is aminomethyl, hydroxymethyl, 1-aminoethyl or 1-methylaminoethyl ;

$R^2$, $R^3$, and $R^6$ are each hydrogen or hydroxy ;

$R^4$ is hydroxy or amino ;

$R^5$ is amino or methylamino ;

$R^7$ is hydroxy or methyl ;

$R^8$ is hydrogen, hydroxymethyl or carbamoyloxymethyl ;

and the dotted line represents the presence or absence of a double bond, with the proviso that if the dotted line represents a double bond, $R^5$ is methylamino.

In the aforementioned general formula (I), acyl group R denotes $C_1$ to $C_6$ acyl, preferably $C_1$ to $C_4$ acyl, e.g. formyl, acetyl, propionyl, butyryl or isobutyryl.

The novel aminoglycoside antibiotic derivatives (I) in this invention include the free bases and salts thereof, particularly non-toxic acid addition salts, for example salts with inorganic acids such as hydrochloric acid, hydrobromic acid, hydroiodic acid, sulfuric acid, phosphoric acid, carbonic acid and the like, and salts with organic acids such as acetic acid, fumaric acid, malic acid, tartaric acid, maleic acid, citric acid, mandelic acid, ascorbic acid, gallic acid, and the like.

Typical examples of the aminoglycosides of the general formula (I) are :

1) 1-N-(3-hydroxy-1-azetin-2-yl)tobramycin
2) 1-N-(3-hydroxy-1-pyrrolin-2-yl)tobramycin
3) 1-N-(3-hydroxy-3,4,5,6-tetrahydropyridin-2-yl)tobramycin
4) 1-N-(3-hydroxy-1-azetin-2-yl)kanamycin A
5) 1-N-(3-hydroxy-1-pyrrolin-2-yl)kanamycin A
6) 1-N-(3-hydroxy-3,4,5,6-tetrahydropyridin-2-yl)kanamycin A
7) 1-N-(3-hydroxy-1-azetin-2-yl)gentamicin $C_1$
8) 1-N-(3-hydroxy-1-pyrrolin-2-yl)gentamicin $C_1$
9) 1-N-(3-hydroxy-3,4,5,6-tetrahydropyridin-2-yl)gentamicin $C_1$

The aminoglycosides of the general formula (I) may be prepared by reacting the known aminoglycosides of the general formula (II) :

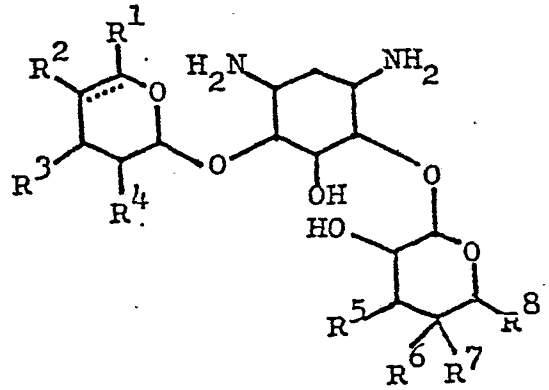

(II)

wherein $R^1$, $R^2$, $R^3$, $R^4$, $R^5$, $R^6$, $R^7$, $R^8$, and the dotted line each have the same meaning as mentioned above, with compounds of the general formula (III) :

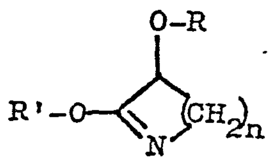

(III)

wherein R and n each have the same meaning as mentioned above; and R' is lower alkyl.

The reaction is usually carried out in a suitable solvent in the presence of a proton source. In carrying out this reaction, an equimolar or excess amount of Compounds (III), preferably about 1.5 to 3 equivalents, is used per one mole of the aminoglycosides. The reaction proceeds well at room temperature and is terminated within a period of 2 to 6 hours. If required, the reaction may be accelerated by carrying it out at elevated temperature. Examples of the solvents employed are alcohols such as methanol, ethanol, and ethylene glycol, dimethylsulfoxide, tetrahydrofuran, dioxane and

the like, and they may be used alone or in combination. As proton sources, acids, preferably weak acids such as carboxylic acids, e.g. acetic acid, are usually employed.

Since the starting aminoglycosides of the general formula (II) have many functional groups (e.g. amino group) besides the 1-amino group which readily react with Compounds of the general formula (III), these may optionally be protected with protecting groups before starting the reaction. Suitable protecting groups include benzyloxycarbonyl of which the benzene ring may be substituted, formyl, t-butyloxycarbonyl, t-amyloxycarbonyl, methoxycarbonyl, ethoxycarbonyl, p-toluenesulfonyl, phthaloyl, m-nitrophenylthio, triphenylmethylthio and the like. These protecting groups may be removed in a conventional manner such as treatment with acids or catalytic hydrogenation after termination of the reaction.

When R of Compounds of the general formula (III) is hydrogen, it is preferable to protect it with a suitable acyl group before the reaction is started.

Representatives of the starting compounds of the general formula (II) and their substituents are shown in Table I.

Table I

| | $R^1$ | $R^2$ | $R^3$ | $R^4$ | $R^5$ | $R^6$ | $R^7$ | $R^8$ | double bond |
|---|---|---|---|---|---|---|---|---|---|
| tobramycin | $CH_2NH_2$ | OH | H | $NH_2$ | $NH_2$ | H | OH | $CH_2OH$ | none |
| kanamycin A | " | " | OH | OH | " | " | " | " | " |
| " B | " | " | " | $NH_2$ | " | " | " | " | " |
| " C | $CH_2OH$ | " | " | " | " | " | " | " | " |
| [illegible]oxykanamycin A | $CH_2NH_2$ | " | H | OH | " | " | " | " | " |
| dideoxykanamycin B (dibekacin) | " | H | " | $NH_2$ | " | " | " | " | " |
| gentamicin $C_1$ | $CH(CH_3)NHCH_3$ | " | " | " | $NHCH_3$ | OH | $CH_3$ | H | " |
| " $C_2$ | CH([illegible])$NH_2$ | " | " | " | " | " | " | " | " |
| " $C_{1a}$ | $CH_2NH_2$ | " | " | " | " | " | " | " | " |
| " B | " | OH | OH | OH | " | " | " | " | " |
| nebramycin factor 4 | " | " | " | $NH_2$ | $NH_2$ | H | OH | $CH_2OCONH_2$ | " |
| nebramycin factor 5' | " | " | H | " | " | " | " | " | " |
| sisomicin | " | H | " | " | $NHCH_3$ | OH | $CH_3$ | H | double bond |

The Compounds of the general formula (III) may be prepared as illustrated in the following reaction scheme :

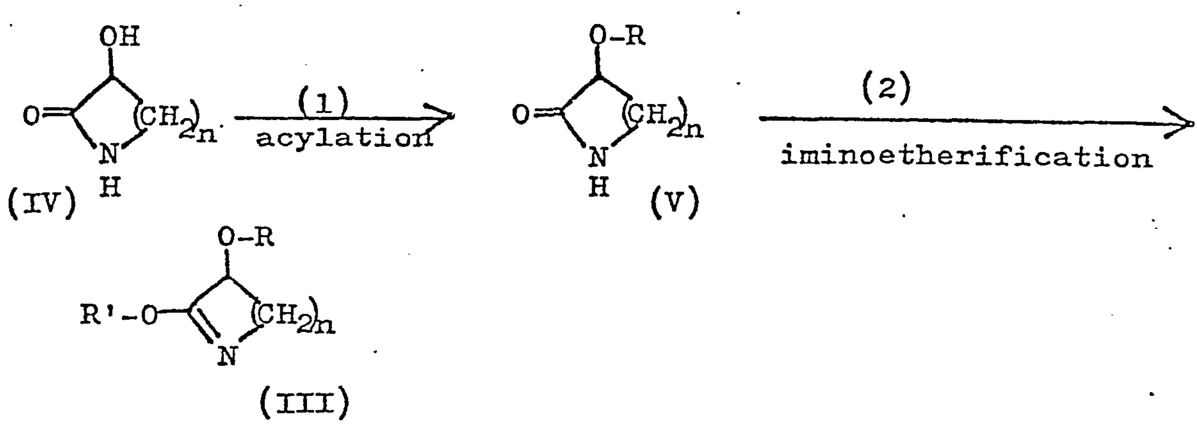


(IV) (V) (III)

wherein R, R' and n each have the same meaning as mentioned above.

The Compounds of the general formula (IV) are known compounds and prepared, for example, in the manner described in Tetrahedron Letters, 2617 (1971).

The acylation may be carried out by treating the starting Compound of the general formula (IV) with an equimolar or excess amount, preferably with 1 to 2 equivalents, of carboxylic acids or the reactive derivatives thereof, based on the acyl R, in a suitable solvent, optionally in the presence of a catalyst such as pyridine or triethylamine.

The reaction proceeds satisfactorily at room temperature but, if required, may be accelerated by heating to 30 to 70°C, preferably 30 to 40°C.
Examples of the reactive derivatives of carboxylic acids

are acid halides, acid azides, acid anhydrides, active esters and the like. Examples of solvents to be used are ethers such as diethyl ether, 1,2-dimethoxyethane, tetrahydrofuran, and dioxane, methylene chloride, dimethylsulfoxide, pyridine, and the like, and these may be used alone or in combination.

This process may be carried out by treating the above acylated intermediates (V) with an equimolar or excess amount, preferably with 1 to 2 equivalents, of an etherifying agent, based on the alkyl to be introduced. Examples of etherifying agents are trialkyloxonium tetrafluoroborate, combined silver oxide and alkyl halide, and the like.

The iminoetherification may also be performed by converting the starting compounds (IV) with a hydrohalogenic acid into the corresponding iminohalide, followed by reaction with a sodium alcoholate of the formula R'ONa.

The aminoglycoside derivatives of the general formula (I) and their salts exhibit an excellent antimicrobial activity. They are in general significantly more active against some species of gram-positive and gram-negative bacteria than the corresponding parent aminoglycosides. The Minimun Inhibitory Concentration (MIC, $\mu$g/ml) of the aminoglycosides of this invention and of the corresponding parent aminoglycosides is indicated in Table II. MIC was determined by the following agar dilution method.

(1) Preparation of bacterial suspensions :

One loopful of a strain of bacteria to be tested on agar slant was inoculated into a medium for growth of inoculum (Trypto Soy Broth; Eiken Chemical Co.) and incubated overnight at 37°C.

(2) Preparation of medium for antibacterial activity test:

A sample solution of aminoglycoside derivative was subjected to several two-fold dilutions with distilled water. These diluted sample solutions were distributed to the Modified Mueller Hinton Agar medium (Nissui-Seiyaku Co.), mixed gently and allowed to solidify.

(3) Determination of MIC values :

One loopful of the bacterial suspension prepared under (1) was placed on the surface of the medium prepared under (2) containing the aminoglycoside derivative in various concentrations at $10^6$ CFU/ml inoculum size. After incubating at 37°C for 18 to 20 hours, the growth of bacteria on the medium was observed in accordance with the standard method of the Japanese Society of Chemotherapy.

Table II

| Compound / Bacteria | MIC (μg/ml) | | |
|---|---|---|---|
| | [A] | [B] | TOB |
| Escherichia coli W-677/JR 762* | 12.5 | 12.5 | 50 |
| Klebsiella pneumoniae K1-168* | 3.1 | 3.1 | 100 |
| Proteus vulgaris TB-615* | 25.0 | 25.0 | 50 |
| Proteus mirabilis TB-617 | 3.1 | 3.1 | 6.2 |
| Pseudomonas aeruginosa PP-6* | 3.1 | 6.2 | >100 |
| Pseudomonas aeruginosa TB-151* | 25.0 | 25.0 | 50.0 |

(Note)

[A] = 1-N-[(S)-(-)-3-hydroxy-1-pyrrolin-2-yl]tobramycin

[B] = 1-N-[(S)-(-)-3-hydroxy-3,4,5,6-tetrahydropyridin-2-yl]tobramycin

TOB = tobramycin

*strains resistant to tobramycin

As seen from Table II, Compounds (I) of this invention are valuable antimicrobial agents effective against various species of strains resistant to the known aminoglycoside, e.g. Klebsiella pneumoniae, Pseudomonas aeruginosa, as well as strains sensitive to the known aminoglycoside. Compounds (I) can also be used as disinfectants to prevent the growth of bacteria in perishables, feedstuffs, or sanitary articles.

Compounds (I) of this invention can be confected in a wide variety of oral or parenteral dosage forms either alone or in admixture with other co-acting substances. The pharmaceutical compositions may consist of a mixture of 0.01 to 99 % of Compounds (I) and a pharmaceutical carrier or carriers which can be solids or liquids in which Compounds (I) are soluble, dispersible, or suspensible. They can be prepared in a unit dosage form. The solid compositions can be produced as tablets, powder, dry syrups, troches, granules, capsules, pills, suppositories, or similar solid preparations. The liquid compositions can be prepared in the form of injectable preparations, ointments, dispersions, inhalants, suspensions, solutions, emulsions, syrups, or elixirs. Any diluents (e.g. starch, sucrose, lactose, calcium carbonate, kaolin); bulking agents (e.g. lactose, sugar, salt, glycine, starch, calcium carbonate, calcium phosphate, kaolin, bentonite, talc, sorbitol); binders (e.g. starch, acacia, gelatin, glucose, sodium alginate, tragacanth, carboxymethylcellulose, sorbitol, polyvinylpyrrolidone); disintegrating agents (e.g. starch, agar, carbonates, sodium laurylsulfate), lubricants (e.g. stearic acid, talc, paraffin, boric acid, silica, sodium benzoate, polyethylene glycol, cacao oil, magnesium sulfate); emulsifying agents (e.g. lecithin, sorbitan monooleate, acacia); suspending agents (e.g. sorbitol,

methylcellulose, glucose, sugar syrup, gelatin, hydroxyethylcellulose, carboxymethylcellulose, aluminum stearate gel, hydrogenated fats) ; solvents (e.g. water, peanut oil, sesame oil, methyl oleate) ; preservatives (e.g. methyl or ethyl p-hydroxybenzoate, sorbic acid), edible coloring agents, aromatic substances, solubilizing agents, buffers, stabilizing agents, dispersing agents, wetting agents, antioxidants, and the like can be used in the conventional manner as far as they do not have an adverse effect on Compounds (I) .

The Compounds (I) of this invention, particularly their sulfates, are readily soluble in water and conveniently used as solutions for intravenous, intramuscular, or subcutaneous injections according to a conventional method. The Compounds (I) can be dissolved in an aqueous or oily solvent for injection to give an injectable solution in an ampoule; in order to preserve the injectable preparation for a long period of time, it is appropriate to make a vial preparation containing the Compounds (I) as crystals, powder, microcrystals, or as a lyophilizate. The vial preparation may be dissolved or suspended in said solvents for injection immediately before use. The preparation may contain said preservatives.

Further, the Compounds (I) of this invention can be used as suppositories, ointments for topical application or use in opthalmology, powders for topical

use, and similar preparations preparable according to well-known methods. The external preparation can contain 0.01 to 99 % of Compounds (I) of this invention together with a necessary amount of pharmaceutical carrier and/or diluent and/or auxiliary agent as stated above.

In order to treat or prevent infections caused by bacteria in humans or domestic animals, the Compounds (I) of this invention are administered in a divided or single dose of 0.01 to 5 g/kg a day if injected, of 0.01 to 10 g/kg a day if administered orally, or of 0.01 to 10 g/kg a day if administered topically at intervals of 3 to 12 hours.

The method can be used to treat or prevent some infectious diseases caused by bacteria sènsitive to the compounds of this invention, e.g. staphylodermia, anthropozoonosis, cystitis, pyelitis, pneumonia, pneumonitis, bronchitis, empyema, naspharyngitis, tonsilitis, rhinitis, dermatitis, pustulosis, abscesses, wound and soft tissue infections, ear infections, osteomyelitis, septicemia, enteritis, urinary tract infections, and pyelonephritis.

Preferably, the Compounds (I) of this invention are given to a patient in the form of a pharmaceutical preparation, e.g. powder, dry syrup, tablets, troches, granules, capsules, pills, suppositories, injections, ointments, dispersions, inhalant, suspensions, solutions, émulsions, syrups and elixirs.

They may be preserved in a unit dosage form e.g. tablets, troches, capsules, injections, vials, granules or powders in a separate container of a package.

The following examples are provided to further illustrate this invention.

Example 1

Preparation of (S)-(-)-3-acetoxy-2-ethoxy-1-pyrroline

(1) To a solution of 1.439 g of (S)-(-)-3-hydroxy-2-pyrrolidinone [P.W.K.Woo, Tetrahedron Letters, 2617 (1971) ; mp. 103.5 - 104.5°C, $[\alpha]_D^{26}$ = -118.8 ± 2.3° (c=0.688, $CHCl_3$)] in 14 ml of pyridine is added 2.8 ml of acetic anhydride, and the mixture is allowed to stand at room temperature for 4 hours and concentrated under reduced pressure at a temperature below 40°C. The residue is crystallized from ether and recrystallized from methylene chloride-n-hexane to give 1.735 g of (S)-(-)-3-acetoxy-2-pyrrolidinone in 86.3 % yield.

mp. 85.5 - 87°C

$[\alpha]_D^{26}$ = -40.1 ± 0.8° (c=1.015, $CHCl_3$)

Elemental Analysis (for $C_6H_9NO_3$)

Calcd (%) : C, 50.35 ; H, 6.29 ; N, 9.79.

Found (%) : C, 50.50 ; H, 6.36 ; N, 9.66.

IR : $\nu_{max}^{Nujol}$ 3170, 3135, 1742, 1715, 1692 $cm^{-1}$.

(2) To a solution of 1.630 g (1.1 equivalents) of triethyloxonium tetrafluoroborate in 10 ml of dry methylene chloride is added a solution of 1.108 g of the above product dissolved in 12 ml of dry methylene chloride under nitrogen atmosphere, and the mixture is allowed to stand at room temperature overnight and cooled. A solution of 1.19 g of potassium carbonate in 1.2 ml of water is added thereto with stirring. The reaction mixture is washed with an ice-cooled aqueous sodium hydrogencarbonate solution, dried over magnesium sulfate, and evaporated to give 1.100 g of 3-acetoxy-2-ethoxy-1-pyrroline in 83 % yield.

bp. 67.5 °C/1.3 mmHg

$[\alpha]_D^{26.5}$ -45.2 ± 0.2° (c=2.41, $CHCl_3$)

Elemental Analysis (for $C_8H_{13}NO_3 \cdot {}^1/_{10}H_2O$)

Calcd (%) : C, 55.54 ; H, 7.69 ; N, 8.10.

Found (%) : C, 55.79 ; H, 7.75 ; N, 8.15.

IR : $\nu_{max}^{CHCl_3}$ 1737, 1653 $cm^{-1}$

Example 2

Preparation of 1-N-[(S)-(-)-3-hydroxy-1-pyrrolin-2-yl] tobramycin

(1) To a solution of 397 mg (2.32 mmoles ; 2.4 equivalents) of (S)-(-)-3-acetoxy-2-ethoxy-1-pyrroline in 28 ml of methanol are added 573 mg (0.96 mmole) of

3,2',6',3"-tetra-N-formyltobramycin and two drops of acetic acid, and the mixture is refluxed for 5 hours, cooled, and concentrated under reduced pressure. The residue is treated with ether, and the resulting precipitate is collected by filtration, washed with ether, dissolved in methanol, and then reprecipitated with addition of ether. The collected precipitate (708 mg) is dissolved in 0.9 ml of water, mixed with 10 ml of concentrated hydrochloric acid -methanol ( volume ratio 22:10 and kept at 35.5°C for 24 hours. The excess amount of hydrochloric acid is neutralized with Amberlite IR-45 (OH type) and the resin is filtered off. The filtrate is evaporated under reduced pressure to dryness to give 747 mg of 1-N-[(S)-(-)-3-hydroxy-1-pyrrolin-2-yl]tobramycin as hydrochloride.

(2) A solution of 200 mg of the above product dissolved in 2 ml of water is adsorbed on a column of 10 ml of Amberlite IRA-400 ($CH_3COOH$ type) and eluted with water. The eluate (20 ml) is evaporated under reduced pressure to give 234 mg of 1-N-[(S)-(-)-3-hydroxy-1-pyrrolin-2-yl] tobramycin as the acetate.

(3) A solution of 234 mg of the above product dissolved in 4 ml of water is adjusted at pH 1.7 with 14 m of 0.1 N sulfuric acid and concentrated to 4 ml under reduc pressure. The residue is treated with 40 ml of ethanol an

the resulting precipitate is collected by filtration, washed with ethanol, dissolved in water, treated with active carbon and lyophilized. The lyophilizate is allowed to stand until the weight becomes constant by absorption of moisture.. Thus, 233 mg of 1-N-[(S)-(-)-3-hydroxy-1-pyrrolin-2-yl] tobramycin sulfate is obtained in 89 % yield.

$[\alpha]_D^{26.0}$ + 71.3 ± 1.0° (c=1.060, $H_2O$)

Elemental Analysis (for $C_{22}H_{42}N_6O_{10} \cdot 2.7H_2SO_4 \cdot 11H_2O$)

Calcd(%) : C, 26.07 ; H, 6.90 ; N, 8.29 ; S, 8.54.

Found(%) : C, 25.79 ; H, 6.66 ; N, 8.44 ; S, 8.57.

NMR : $\delta_{ppm}^{D_2O}$ 6.48d, 5.83d, 5.67(t, J=8.0Hz).

Example 3

Preparation of (S)-(-)-3-acetoxy-2-ethoxy-3,4,5,6-tetrahydropyridine

(1) To a solution of 460 mg of (S)-(-)-3-hydroxy-2-piperidone [A. Hunter, Biochemical Journal, 35, 1298 (1941); mp. 170 - 171°C, $[\alpha]_D^{26}$ = -3.0 ± 0.1° (c=7.027, $H_2O$)] in 6 ml of pyridine is added 2 ml of acetic anhydride. The mixture is allowed to stand at room temperature overnight and then concentrated under reduced pressure. The residue is crystallized from ether and then recrystallized from ether to give 560 mg of (S)-(-)-3-acetoxy-2-piperidone as pillar crystals in 89 % yield.

mp. 77 - 78°C

$[\alpha]_D^{26}$ = -20.5 ± 0.2° (c=3.049, $CHCl_3$)

Elemental Analysis (for $C_7H_{11}NO_3$)

Calcd(%) : C, 53.49 ; H, 7.05 ; N, 8.91.

Found(%) : C, 53.28 ; H, 7.01 ; N, 8.79.

IR : $\nu_{max}^{Nujol}$ 3260, 1735, 1700, 1501 $cm^{-1}$.

(2) To a solution of 3.0 g of the above product dissolved in 20 ml of dry methylene chloride is added a solution of 3.99 g (1.1 equivalents) of triethyloxonium tetrafluoroborate in 40 ml of dry methylene chloride. The mixture is allowed to stand at room temperature overnight, cooled, mixed with a solution of 2.692 g of potassium carbonate in 2.7 ml of water with stirring, washed with an ice-cooled aqueous sodium hydrogencarbonate solution, dried over magnesium sulfate, and evaporated. The resulting syrupy residue is further evaporated to dryness under reduced pressure to give 2.68 g of (S)-(-)-3-acetoxy-2-ethoxy-3,4,5,6-tetrahydropyridine in 76 % yield.

bp. 67 - 67.5°C/1.3 mmHg

$[\alpha]_D^{26.5}$ =-128.0 ± 0.5° (c=3.123, $CHCl_3$)

Elemental Analysis (for $C_9H_{15}NO_3 \cdot {}^1/_5 H_2O$)

Calcd(%) : C, 57.24 ; H, 8.21 ; N, 7.42.

Found(%) : C, 57.47 ; H, 8.13 ; N, 7.42.

IR : $\nu_{max}^{CHCl_3}$ 1734, 1678 $cm^{-1}$.

Example 1

Preparation of 1-N-[(S)-(-)-3-hydroxy-3,4,5,6-tetrahydropyridin-2-yl]tobramycin

(1) To a solution of 204 mg (1.1 mmoles) of (S)-(-)-3-acetoxy-2-ethoxy-3,4,5,6-tetrahydropyridine in 16 ml of methanol are added 299 mg (0.5 mmole) of 3,2',6',3"-tetra-N-formyltobramycin and three drops of acetic acid. The mixture is then refluxed for 3 hours, cooled, concentrated under reduced pressure, and treated with ether. The resulting precipitate is collected by filtration, washed with ether, dissolved in methanol and reprecipitated with addition of ether. A solution of 385 mg of the precipitate in 0.8 ml of water is mixed with 5.41 ml of concentrated hydrochloric acid (volume ratio 22:109) and kept at 35.°C for 24 hours. The excess amount of hydrochloric acid is neutralized with Amberlite IR-45 (OH type) and the resin is filtered off. The filtrate is evaporated under reduced pressure to give 410 mg of 1-N-[(S)-(-)-3-hydroxy-3,4,5,6-tetrahydropyridin-2-yl]tobramycin as hydrochloride.

(2) A solution of 248 mg of the above product dissolved in 4 ml of water is adsorbed on a column of 10 ml of Amberlite IRA-400 ($CH_3COOH$ type) and eluted with water. The eluate is evaporated under reduced pressure to give 258 mg of 1-N-[(S)-(-)-3-hydroxy-3,4,5,6-tetrahydropyridin-2-yl]tobramycin as the acetate.

(3) To a solution of 258 mg of the above product dissolved in 5 ml of water is added 16.7 ml of 0.1 N sulfuric acid, and the mixture is concentrated to 2 ml under reduced pressure and treated with 20 ml of ethanol. The resulting precipitate is collected by filtration, washed with ethanol, dissolved in water, treated with active carbon and lyophilized. The lyophilizate is allowed to stand until the weight becomes constant with absorption of moisture. Thus, 311 mg of 1-N-[(S)-(-)-3-hydroxy-3,4,5,6-tetrahydropyridin-2-yl]tobramycin sulfate is obtained in 94 % yield.

$[\alpha]_D^{27} = + 67.2 \pm 1.0°$ (c=1.027, $H_2O$)

Elemental Analysis (for $C_{23}H_{44}N_6O_{10} \cdot 2.5H_2SO_4 \cdot 9.5H_2O$)

Calcd(%) : C, 28.16 ; H, 6.99 ; N, 8.57 ; S, 8.17.

Found(%) : C, 28.14 ; H, 6.93 ; N, 8.46 ; S, 8.06.

NMR : $\delta_{ppm}^{D_2O}$: 6.45d, 5.78d, 5.08br.

What is claimed is:

1. Aminoglycoside derivatives of the general formula I

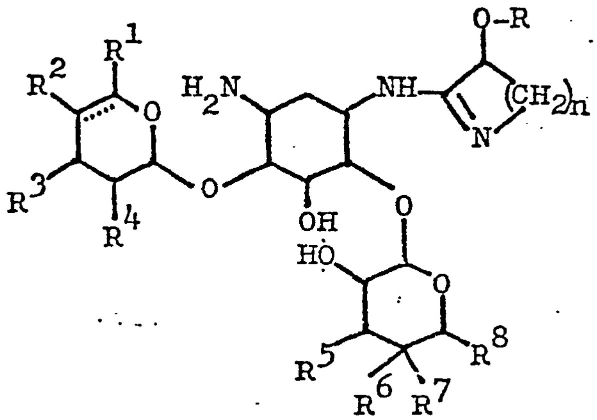

(I)

wherein R is hydrogen or acyl ;

n is an integer of 1 to 3 ;

$R^1$ is aminomethyl, hydroxymethyl, 1-aminoethyl, or 1-methylaminoethyl ;

$R^2$, $R^3$ and $R^6$ are each hydrogen or hydroxy ;

$R^4$ is hydroxy or amino ;

$R^5$ is amino or methylamino ;

$R^7$ is hydroxy or methyl ;

$R^8$ is hydrogen, hydroxymethyl or carbamoyloxymethyl;

and the dotted line represents the presence or absence of a double bond, with the proviso that if the dotted line represents a double bond, $R^5$ is methylamino,

and their salts with acids.

2. A compound claimed in claim 1, wherein n is an integer of 2 or 3.

3. A compound claimed in claim 1, wherein R is hydrogen.

4. A compound claimed in claim 1, wherein $R^8$ is hydrogen or hydroxymethyl.

5. A compound claimed in claim 1, wherein $R^1$ is aminomethyl or hydroxymethyl and $R^8$ is hydrogen or hydroxymethyl.

6. A compound claimed in claim 1, wherein $R^1$ is aminomethyl or hydroxymethyl and $R^8$ is hydroxymethyl.

7. A compound claimed in claim 1, wherein $R^1$ is aminomethyl, $R^2$ is hydroxy, $R^5$ is amino, $R^6$ is hydrogen, $R^7$ is hydroxy, and $R^8$ is hydroxymethyl.

8. A compound claimed in claim 1, namely 1-N-(3-hydroxy-1-pyrrolin-2-yl)tobramycin.

9. A compound claimed in claim 1, namely 1-N-(3-hydroxy-3,4,5,6-tetrahydropyridin-2-yl)tobramycin.

10. A process for preparing the compounds claimed in claim 1, which comprises reacting an aminoglycoside of the general formula (II)

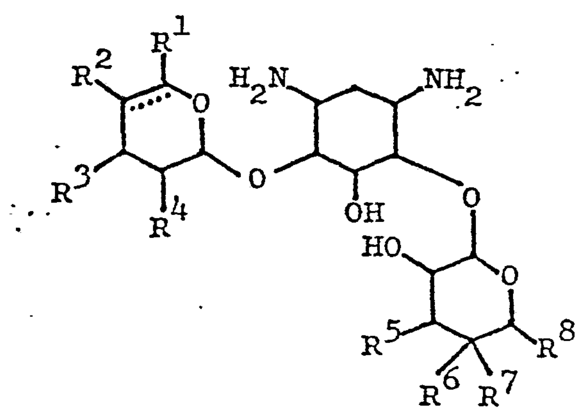

(II)

wherein $R^1$, $R^2$, $R^3$, $R^4$, $R^5$, $R^6$, $R^7$, $R^8$, and the dotted line each have the same meaning as in claim 1, of which the functional groups other than the 1-amino group are optionally protected, with a compound of the general formula (III)

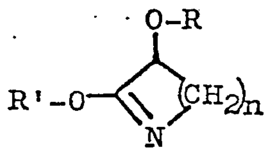

(III)

wherein R, R' and n each have the same meaning as in claim 1, followed, if necessary, by deprotection of the functional groups and, if desired, converting the aminoglycoside derivative obtained into its salt by treatment with an inorganic or organic acid.

11. A process claimed in claim 10, wherein the reaction is carried out in the presence of a proton source.

12. A process claimed in claim 10, wherein the reaction is carried out at room temperature.

13. Pharmaceutical compositions comprising an effective amount of a compound claimed in claim 1 and a pharmaceutically acceptable carrier and/or diluent and/or auxiliary agent.

0009670

Application number

EP 79 10 3380

European Patent Office

# EUROPEAN SEARCH REPORT

| DOCUMENTS CONSIDERED TO BE RELEVANT | | | CLASSIFICATION OF THE APPLICATION (Int. Cl. ³) |
|---|---|---|---|
| Category | Citation of document with indication, where appropriate, of relevant passages | Relevant to claim | |
| | FR - A - 2 369 295 (SHIONOGI)<br>* Pages 26-29 *<br>-- | 1,13 | C 07 H 15/22<br>A 61 K 31/70//<br>C 07 D 207/26<br>C 07 D 207/24<br>C 07 D 211/76 |
| | FR - A - 2 240 015 (SCHERICO)<br>* Pages 116-126 *<br>-- | 1,13 | |
| D | CARBOHYDRATE RESEARCH, 28 (1973) 263-280, Elsevier Scientific Publishing Company Amsterdam (NL)<br>T.H. HASKELL et al.: "The preparation and biological activity of novel amino acid analogs of butirosin"<br>* Pages 263, 264, 267, no. 52 *<br>-- | 1,13 | TECHNICAL FIELDS SEARCHED (Int.Cl. ³)<br>C 07 H 15/22<br>A 61 K 31/70 |
| P | EP - A - 0 002 835 (SHIONOGI)<br>* Pages 1-3 *<br>---- | 1,13 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 28-11-1979 | VERHULST |